# EUROPEAN PATENT SPECIFICATION

(11) **EP 2 433 621 B1**
(45) Date of publication and mention of the grant of the patent: **01.05.2013**
(21) Application number: 11192895.8
(22) Date of filing: 07.09.2007
(51) Int. Cl.: A61K 9/28

(54) **Rapidly disintegrating dosage form**
Schnell zerfallende Darreichungsform
Forme de dosage à désintégration rapide

(30) Priority: 15.09.2006 US 845123 P
(43) Date of publication of application: 28.03.2012
(62) Divisional of application: 07804907.9
(73) Proprietor: Capsugel Belgium NV, 2880 Bornem (BE)
(72) Inventor: Murachanian, Dennis Carl, Tinton Falls, NJ07724 (US); Kirk, John Richard, McKeesport, PA15132 (US)
(74) Representative: Krishnan, Sri

(56) References cited:
- WO-A1-2006/099618
- US-A- 2 052 376
- US-A- 4 503 031
- US-A1- 2005 152 970

## Description

### TECHNICAL FIELD

The invention relates generally to dosage forms, and more particularly, to a rapidly disintegrating dosage form.

### BACKGROUND OF THE INVENTION

Film-coated tablets represent a preferred dosage form for the oral delivery of medicaments, vitamins, and other compounds. Their elongate shape and film-coated surfaces facilitate swallowing while their tablet core, which may be a molded or compressed tablet, provide a level of tamper resistance additional to that of the film coating itself. In addition, the use of colored, patterned, or labeled film coatings allows easy identification of the pharmaceutical or other substance being delivered. Such tablets are, for example known from US-A-4,503,031. US 4,503,031 relates to super-fast-starting sustained release medicinal tablet. US 2,052,376 relates to a insoluble pill or tablet for internal medicinal uses.

However, film-coated tablets are not without defects. For example, rapid release of the medicament, vitamin, etc. contained within the tablet is hindered by the film coating. That is, while a film coating (often containing a gelatin) employed in such a tablet is necessarily dissolvable or disintegrable, the film coating must at least partially dissolve or disintegrate before the tablet core can dissolve or disintegrate, thereby releasing its medicament, vitamin, etc. The use of more easily dissolving film coatings is an unsatisfactory solution, due to the increased likelihood that the dosage form will be compromised by high humidity, contact with a small amount of water, or other environmental conditions.

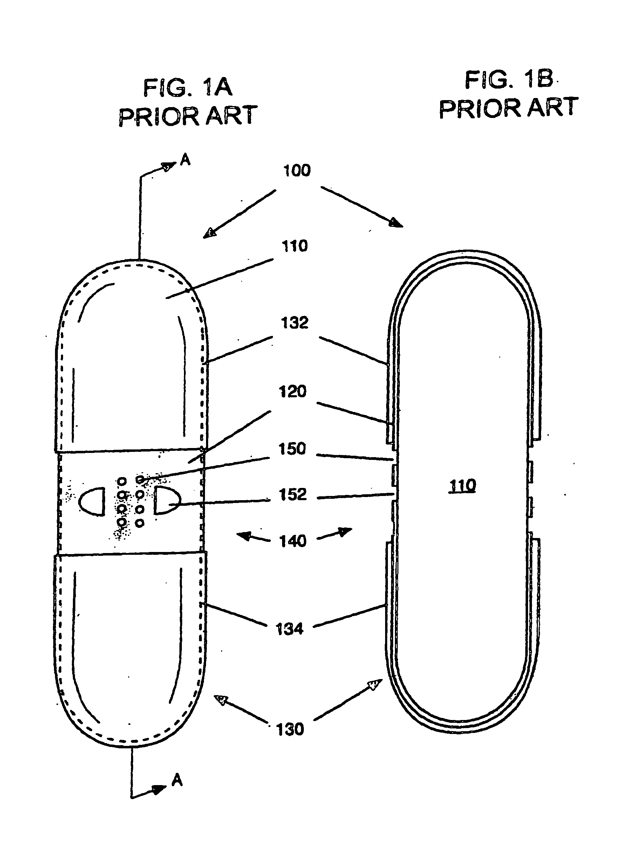

Proposed solutions are disclosed in U.S. Patent Application Publication Nos. 2005/0152970 and 2005/0152971. Referring now to FIGS. 1 A-B, the dosage forms 100 of 2005/0152970 and 2005/0152971 comprise a tablet core 110 coated with a polymeric subcoating 120 and a film coating 130 comprising a pair of gelatinous end caps 132, 134. In FIG. 1A, it can be seen that gelatinous end caps 132, 134 are sized such that, together, they do not fully cover tablet core 110, resulting in a gap 140 at approximately the middle of table core 110. Into subcoating 120 are formed one or more openings 150, 152, such that tablet core 110 is partially exposed to liquids and digestive enzymes, thereby resulting in a more rapid release of the contents of tablet core 110 than would result from a dosage form lacking such openings 150, 152. FIG. 1 B shows a cross-sectional view of dosage form 100 of FIG. 1A taken along line A, perpendicular to a longitudinal axis of dosage form 100.

Such dosage forms, however, are also unsatisfactory. First, the process for producing such a dosage form is far more complex, and therefore expensive, than other processes (i.e., the tablet core 110 includes two coatings - polymeric subcoating 120 and gelatinous film coating 130; openings 150, 152 must be formed into subcoating 120, etc.). In addition, any improved rate or extent of dissolution/disintegration of tablet core 110 is limited to the relatively small exposed surface area of tablet core 110 afforded by openings 150, 152. That is, openings 150, 152 cannot expose too large a surface area of tablet core 110 or the benefits of a film-coated tablet, such as those described above, will be lost.

To this extent, a need exists for a film-coated tablet dosage form exhibiting improved dissolvability and/or disintegrability that does not suffer from the defects of known dosage forms.

### SUMMARY OF THE INVENTION

The invention provides a method for improving the rate of disintegration and/or the extent of disintegration in a tablet dosage form, the method comprising: forming in a surface of a tablet at least one groove, wherein the tablet dosage form is in elongated cylindrical shape and the at least one groove increases a surface area of the tablet wherein the at least one groove is elongated and is oriented substantially parallel to a longitudinal axis of the tablet.

In another embodiment, the tablet is a compressed tablet or a molded tablet.

In yet another embodiment, forming includes forming at least two grooves in the surface of the tablet oriented substantially parallel to each other.

The illustrative aspects of the present invention are designed to solve the problems herein described and other problems not discussed, which are discoverable by a skilled artisan.

### BRIEF DESCRIPTION OF THE DRAWINGS

These and other features of this invention will be more readily understood from the following detailed description of the various aspects of the invention taken in conjunction with the accompanying drawings that depict various embodiments of the invention, in which: FIGS. 1A-B show side and cross-sectional views, respectively, of a known dosage form.

FIGS. 2A-C show partial side cross-sectional, side, and cross-sectional views, respectively, of a dosage form according to an embodiment of the invention.

FIGS. 3A-C show partial side cross-sectional, side, and cross-sectional views, respectively, of a dosage form according to an alternative embodiment of the invention.

FIGS. 4A-C show partial side cross-sectional, side, and cross-sectional views, respectively, of a dosage form according to another alternative embodiment of the invention.

It is noted that the drawings of the invention are not to scale. The drawings are intended to depict only typical aspects of the invention, and therefore should not be considered as limiting the scope of the invention. In the drawings, like numbering represents like elements between the drawings.

### DETAILED DESCRIPTION

As indicated above, the invention provides a rapidly disintegrating dosage form comprising a partially-coated tablet having at least one groove along its surface, such that a portion of the groove extends beneath the coating.

As used herein, the terms "disintegrate," "disintegrating," and "disintegrable" shall include "dissolve," "dissolving," and "dissolvable."

As used herein, "dosage form" shall include any solid, semi-solid, or liquid composition containing a predetermined dose of an ingredient, such as a medicament, vitamin, or other active ingredient. Dosage forms according to the present invention are, for example, solid objects, although they may contain semi-solid or liquid components. More particularly, dosage forms according to the invention include solid tablet cores. Such tablet cores may be molded or compressed tablets, as known in the pharmaceutical arts, and are in elongated cylindrical shapes, particularly somewhat flattened to form an ovoid shape.

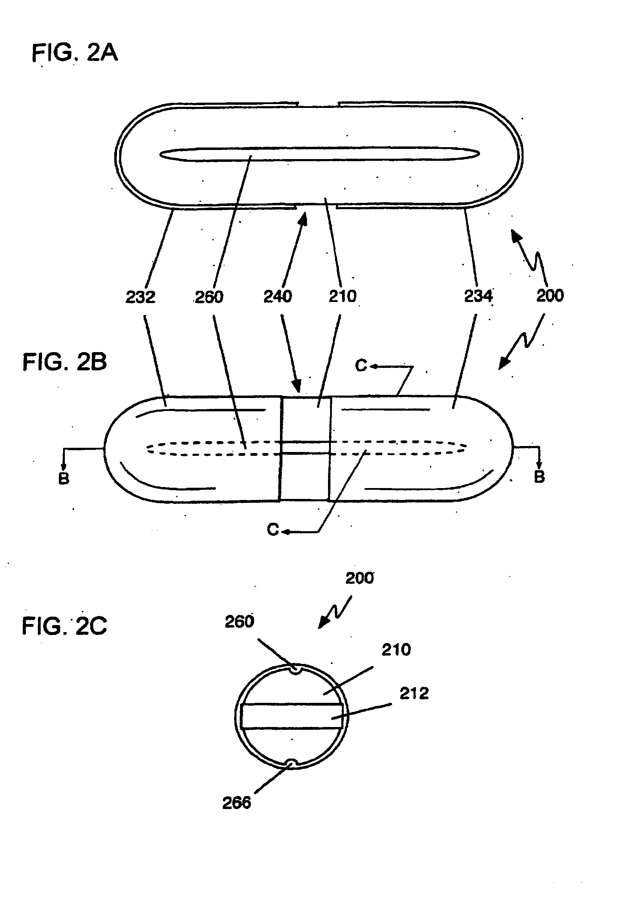

Referring now to FIGS. 2A-C, various views of an illustrative embodiment of a dosage form 200 according to the invention are shown. FIG. 2A shows a partial side cross-sectional view of dosage form 200 taken along line B, perpendicular to a longitudinal axis of FIG. 2B. As shown in FIGS. 2A-B, dosage form 200 comprises a tablet core 210 having at least one groove 260 on its surface and a pair of coating ends 232, 234 covering opposite ends of tablet core 210. In FIG. 2A, only coating ends 232, 234 are shown in cross-section (i.e., a surface of tablet core 210 is shown rather than its cross section). As shown in FIGS.2A-B, coating ends 232, 234 are sized such that less than all of tablet core 210 is covered, thereby providing a gap 240 exposing a portion of tablet core 210.

In one particular embodiment, groove 260 is an elongated groove oriented substantially parallel to a longitudinal axis of tablet core 210. In any case, at least a portion of groove 260 extends beneath at least one coating ends 232, 234. Thus, a passage is formed from an area outside dosage form 200, into groove 260, and beneath coating end 232, 234. Such a configuration greatly improves the rate and/or extent of disintegration of tablet core 210 by providing an increased surface area of tablet core 210 upon which liquids and/or digestive enzymes may immediately act after ingestion of dosage form 200. Specifically, such a configuration provides access to a surface area beneath coating ends 232, 234, which otherwise (and as in known dosage forms) is unavailable to liquids and/or digestive enzymes until coating ends 232, 234 have, at least partially, disintegrated or dissolved.

Any number of grooves may be provided on a surface of tablet core 210. FIG. 2C shows a cross-sectional view of dosage form 200 taken along line C, in a plane perpendicular to a longitudinal axis of dosage form 200. As can be seen, dosage form 200 actually includes a pair of grooves 260, 266, each groove being oppositely disposed in relation to the other. Preferably, a dosage form according to the invention includes at least two grooves distributed evenly (e.g., opposite each other, at equal intervals around a circumference of the tablet core, etc.), such that the tablet core disintegrates evenly. In FIG. 2C, tablet core 210 is shown having a belly band 212, as is typically found on compressed tablets, although this is not essential.

The number of grooves employed will depend, in part, upon the desired rate of disintegration and the size of each groove. Typically, for a tablet core 210 having a length between about 18 mm and about 20 mm, such as about 19 mm, and a diameter between about 5 mm and about 7 mm, such as about 6 mm, each of the two grooves 260, 266 will have a length of between about 14.5 mm and about 16.6 mm, such as about 15.5 mm, a width of between about 0.5 mm and about 2.5 mm, such as about 1.5 mm, and a depth of between about 0.5 mm and about 0.7 mm, such as about 0.6 mm. As will be recognized, the presence of a groove on a surface of tablet core increases the surface area of the tablet core, thereby increasing the rate and/or extent of disintegration of the tablet core as compared to a tablet core not having a groove.

As noted, the groove dimensions provided above correspond to a tablet having a length of about 19 mm and a diameter of about 6 mm. Tablets of other sizes may also be used, of course, with the dimensions of their grooves being greater or less than the dimensions provided above. Typically, tablets employed in practicing the present invention will have lengths between about 10 mm and about 22 mm.

To achieve a rate and/or extent of disintegration substantially the same as the tablet core described above, a greater number of grooves (e.g., three, four, five, six, etc.) of smaller size may be employed. Contrarily, use of a greater number of similarly- sized grooves will yield a disintegration rate and/or extent greater than such a tablet core. In any case, dosage forms according to the present invention will typically include tablet cores having grooves with lengths between about 12 mm and about 17 mm, widths between about 0.5 mm and about 2.5 mm, and depths between about 0.2 mm and about 0.7 mm.

Coating ends 232, 234 may be of any known or later developed material suitable for use in pharmaceutical or nutritional dosage forms. Particular examples of coating ends 232, 234 are gelatinous (e.g., comprise a gelatin, type A (derivative of acid-treated raw materials) or type B (derivative of alkali-treated raw materials)), although other materials are also suitable, including, for example, cellulose ethers, such as hydroxypropylmethylcellulose (HPMC); starches, including waxy maize starch, tapioca dextrin, and derivatives thereof; carrageenan; and polymers or copolymers of (meth)acrylic acids and derivatives thereof.

Due to the unique configuration of dosage form 200 and its improved rate and/or extent of disintegration of tablet core 210, described above, it is possible to utilize materials having low rates of disintegration in coating ends 232, 234. In fact, in some embodiments of the invention (e.g., dosage forms having many and/or large grooves), it is possible to utilize non-disintegratable and/or nonsoluble materials in coating ends 232, 234, allowing tablet core 210 to disintegrate within coating ends 232, 234 as liquids and/or digestive enzymes travel under coating ends 232, 234 through groove 260.

In any case, coating ends may be applied by any known or later-developed method or technique capable of leaving the portion of the groove beneath the coating end unfilled. That is, any method or technique for applying the coating ends may be employed, provided that a space under the coating end is maintained within the groove. Most dip coating methods, for example, would be inappropriate in practicing the present invention, as such methods would tend to coat the grooves as well as the ungrooved surface of the tablet core. Suitable methods, however, include, for example, those used by the Capsugel division of Pfizer, Inc. in forming their Press-Fit® Gelcaps. These methods are described in U.S. Patent Nos. 5,317,849; 5,460,824; 5,464,631 ; 5,511,361 ; 5,795,588; 5,609,010; 6,080,426 and 6,245,350, and European Patent Application Publication No. 1396263.

Dosage forms according to the present invention provide a number of additional benefits over known dosage forms. First, the groove(s) provided in the tablet surface reduce a diameter of the tablet, facilitating easier and more accurate splitting of the tablet, should such splitting be desirable. For example, the present invention includes an uncoated tablet core having at least one groove in its surface, such that the tablet core may be more accurately divided along the groove using less force than would be necessary to divide a tablet core not having a groove in its surface.

Second, in the case that a dosage form according to the present invention is to include coating ends, as described above, the presence of one or more grooves along a surface of the tablet core facilitates the placement of coating ends over the ends of the tablet core by providing a passage for air contained within the coating ends, thereby reducing the resistance encountered while applying the coating ends. The encapsulation methods described in European Patent Application Publication No.1396263 may also be employed to form an "interim" film coating onto the grooved or ungrooved tablet cores, but prior to the formation of grooves and/or application of the coating ends. Such an interim coating improves adhesion between the tablet core and the coating ends and also reduces the likelihood that the tablet core will be contaminated (e.g., by dust) before or during the application of the coating ends, as the interim coating typically has a smoother surface less likely to retain airborne contaminates.

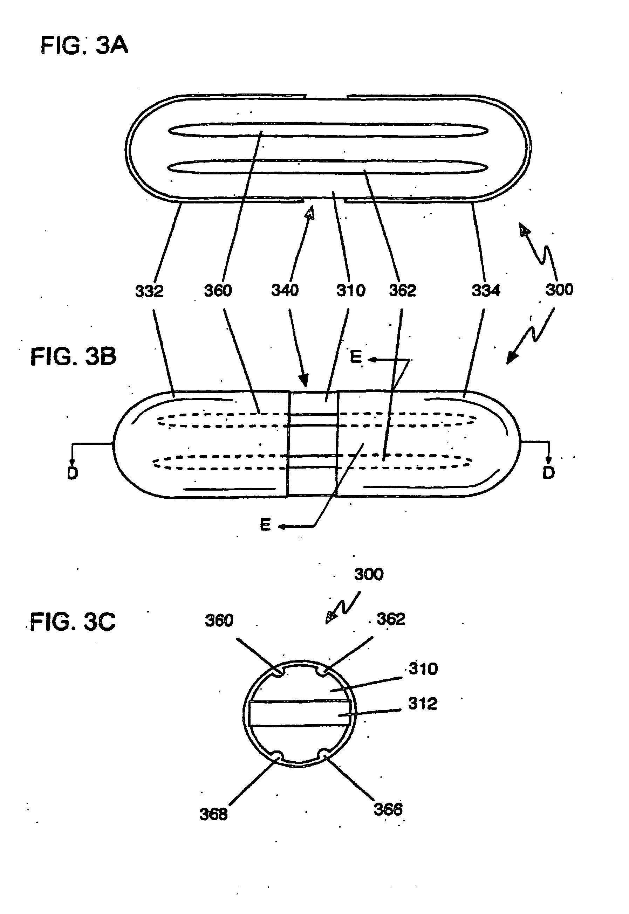

Referring now to FIGS. 3A-C, various views of a dosage form 300 according to an alternative embodiment of the invention are shown. FIG. 3A is a partial side cross- sectional view of the dosage form 300 of FIG. 3B taken along line D, perpendicular to a longitudinal axis of the dosage form.

FIG. 3C is a cross-sectional view of dosage form 300 taken along line E. Here, tablet core 310 includes four grooves 360, 362, 366, 368 arranged in two opposing pairs (i.e., 360 and 366; 362 and 368). Other arrangements are possible, of course, and, as explained above, while a particular groove embodiment is given having evenly distributed around a circumference of tablet core 310, this is not essential.

In the case that tablet core 310 has approximately the same dimensions of tablet core 210, described above with respect to FIGS. 2A-C, each groove 360, 362, 366, 368 may have a length of between about 13 mm and about 15 mm, a width of between about 0.9 mm and about 1.3 mm, and a depth of between about 0.4 mm and about 0.6 mm.

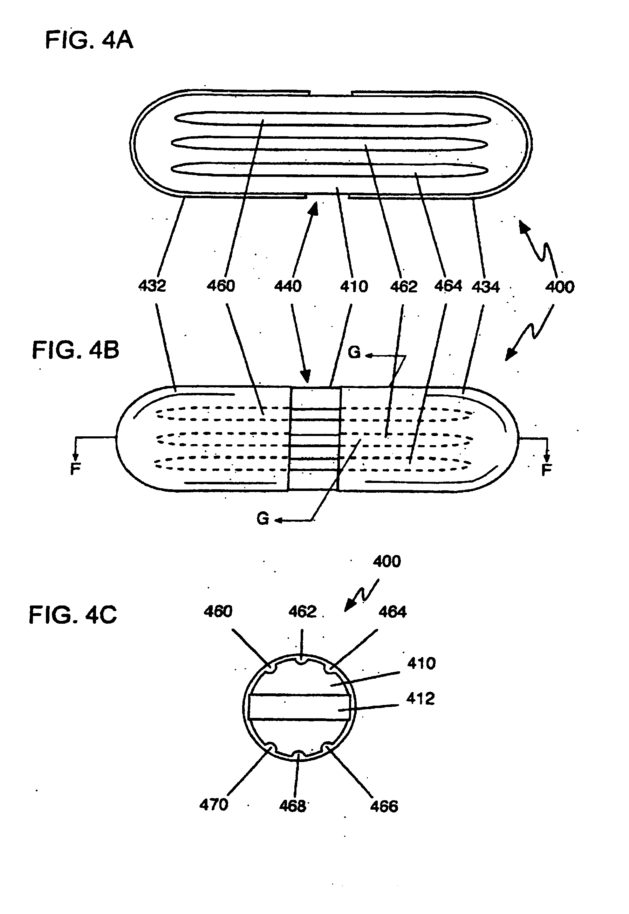

FIGS. 4A-B show various views of yet another alternative embodiment of a dosage form 400 according to the invention. FIG. 3A is a partial side cross-sectional view of dosage form 400 of FIG. 4B taken along line F.

As can be seen in FIG. 4C, a cross-sectional view along line G of FIG. 4B, tablet core 410 includes six grooves 460, 462, 464, 466, 468, 470 arranged in three opposing pairs (i.e., 460 and 466; 462 and 468; 464 and 470). Other arrangements are possible, of course, and, as explained above, while a particular embodiment may have grooves evenly distributed around a circumference of tablet core 410, this is not essential.

While the alternative embodiments described above and shown in FIGS. 2A-C, 3A-C, and 4A-C show grooves extending beneath both coating ends, it should be recognized that this is not essential. That is, one or more grooves may extend beneath one coating end but not the other coating end. In any case, the exposed portion of a groove preferably comprises between about 1 % and about 75%, more preferably between about 2.5% and about 50%, and most preferably between about 5% and about 20% of the portion of the groove beneath the coating end(s). That is, the portion of a groove beneath the coating end(s) preferably comprises between about 10,000% and about 133%, more preferably between about 4000% and about 200%, and most preferably between about 2000% and about 500% of the exposed portion of the groove.

Similarly, while the grooves shown in FIGS. 2A-C, 3A-C, and 4A-C are shown having an orientation substantially parallel with a longitudinal axis of the tablet core, other arrangements are possible, of course. For example, grooves may be oriented in a spiral around the tablet core, extending from a point closer to one end to a point closer to the other end. Such an embodiment may be useful, for example, where the tablet core does not include a belly band (e.g., 412 in FIG. 4C).

The dosage forms of the present invention may, for example, comprise a pharmaceutically or agrochemically active composition. Furthermore, comprised in the dosage form can, for example, be a foodstuff or a dyestuff composition. In case that a dosage form of the present invention contains a pharmaceutical composition, particularly an effective amount of such pharmaceutical composition, the active substance may, for example, be selected from the group consisting of: betamethasone, thioctic acid, sotalol, salbutamol, norfenefrine, silymarin, dihydroergotamine, buflomedil, etofibrate, indomethacin, oxazepam, acetyldigitoxins, piroxicam, haloperidol, isosorbide mononitrate, amitriptyline, diclofenac, nifedipine, verapamil, pyritinol, nitrendipine, doxycycline, bromhexine, methylprednisolone, clonidine, fenofibrate, allopurinol, pirenzepine, levothyroxine, tamoxifen, metildigoxin, o-(B-hydroxyethyl)-rutoside, propicillin, aciclovirmononitrate, paracetamolol, naftidrofuryl, pentoxifylline, propafenone, acebutolol, 1 -thyroxin, tramadol, bromocriptine, loperamide, ketofinen, fenoterol, ca-dobesilate, propranolol, minocycline, nicergoline, ambroxol, metoprolol, B- sitosterin, enalaprilhydrogenmaleate, bezafibrate, isosorbide dinitrate, gallopamil, xantinolnicotinate, digitoxin, flunitrazepam, bencyclane, depanthenol, pindolol, lorazepam, diltiazem, piracetam, phenoxymethylpenicillin, furosemide, bromazepam, flunarizine, erythromycin, metoclopramide, acemetacin, ranitidine, biperiden, metamizol, doxepin, dipotassiumchlorazepat, tetrazepam, estramustinephosphate, terbutaline, captopril, maprotiline, prazosin, atenolol, glibenclamid, cefaclor, etilefrin, cimetidine, theophylline, hydromorphone, ibuprofen, primidone, clobazam, oxaceprol, medroxyprogesterone, flecainide, Mg-pyridoxal-5-phosphateglutaminate, hymechromone, etofyllineclofibrate, vincamine, cinnarizine, diazepam, ketoprofen, flupentixol, molsidomine, glibornuride, dimethindene, melperone, soquinolol, dihydrocodeine, clomethiazole, clemastine, glisoxepid, kallidinogenase, oxyfedrine, baclofen, carboxymethylcystsin, thioredoxin, betahistine, 1 - tryptophan, myrtol, bromelain, prenylamine, salazosulfapyridine, astemizole, sulpiride, benzerazid, dibenzepin, acetylsalicylic acid, miconazole, nystatin, ketoconazole, sodium picosulfate, colestyramate, gemfibrozil, rifampin, fluocortolone, mexiletine, amoxicillin, terfenadine, mucopolysaccharidpolysulfuric acid, triazolam, mianserin, tiaprofensaure, ameziniummethylsulfate, mefloquine, probucol, quinidine, carbamazepine, Mg-1 - aspartate, penbutolol, piretanide, amitriptyline, caproteron, sodium valproinate, mebeverine, bisacodyl, 5-amino-salicyclic acid, dihydralazine, magaldrate, phenprocoumon, amantadine, naproxen, carteolol, famotidine, methyldopa, auranofine, estriol, nadolol, levomepromazine, doxorubicin, medofenoxat, azathioprine, flutamide, norfloxacin, fendiline, prajmaliumbitartrate, aescin acromycin, anipamil, benzocaine, B- carotene, cloramphenicol, chlorodiazepoxid, chlormadinoneacetate, chlorothiazide, cinnarizine, clonazepam, codeine, dexamethasone, dicumarol, digoxin, drotaverine, gramicidine, griseofulvin, hexobarbital hydrochlorothiazide, hydrocortisone, hydroflumethiazide, ketoprofen, Ionetil, medazepam, mefruside, methandrostenolone, sulfaperine, nalidixic acid, nitrazepam, nitrofurantoin, estradiol, papaverine, phenacetin, phenobarbital, phenylbutazone, phenytoin, prednisone, reserpine, spironolactine, streptomycin, sulfamethizole, sulfamethazine, sulfamethoxoazole, sulfamethoxydiazinon, sulfathiazole, sulfisoxazole, testosterone, tolazamide, tolbutamide, trimethoprim, tyrothricin, and mixtures thereof.

## Claims

1. A method for improving the rate of disintegration and/or the extent of disintegration in a tablet dosage form, the method comprising:
forming in a surface of a tablet at least one groove, wherein the at least one groove increases a surface area of the tablet wherein the the tablet dosage form is in elongated cylindrical shape and at least one groove is elongated and is oriented substantially parallel to a longitudinal axis of the tablet.

2. The method of claim 1, wherein the tablet is a compressed tablet or a molded tablet.

3. The method of claim 1, wherein forming includes forming at least two grooves in the surface of the tablet oriented substantially parallel to each other.

## Patentansprüche

1. Verfahren zur Verbesserung der Zerfallsgeschwindigkeit und/oder dem Ausmaß des Zerfalls in einer Tablettendosierungsform, wobei das Verfahren Folgendes umfasst:
Ausbilden von mindestens einer Einkerbung in einer Oberfläche einer Tablette, wobei die mindestens eine Einkerbung einen Oberflächenbereich der Tablette vergrößert, wobei die Tablettendosierungsform in einer langgestreckten zylindrischen Form vorliegt und mindestens eine Einkerbung langgestreckt und im Wesentlichen parallel zu einer Längsachse der Tablette ausgerichtet ist.

2. Verfahren gemäß Anspruch 1, wobei die Tablette eine komprimierte Tablette oder eine geformte Tablette ist.

3. Verfahren gemäß Anspruch 1, wobei das Formen das Ausbilden von mindestens zwei Einkerbungen in der Oberfläche von der Tablette umfasst, die im Wesentlichen parallel zueinander ausgerichtet sind.

## Revendications

1. Procédé pour améliorer la vitesse de désintégration et/ou l'étendue de désintégration dans une forme de dosage en cachet, le procédé comprenant :
la formation dans une surface d'un cachet d'au moins une rainure, dans laquelle l'au moins une rainure augmente une superficie du cachet dans lequel la forme de dosage en cachet a une forme cylindrique allongée et au moins une rainure est allongée et est orientée sensiblement parallèlement à un axe longitudinal du cachet.

2. Procédé selon la revendication 1, dans lequel le cachet est un cachet comprimé ou un cachet moulé.

3. Procédé selon la revendication 1, dans lequel la formation inclut la formation d'au moins deux rainures dans la surface du cachet orientées sensiblement parallèlement l'une à l'autre.
